# EUROPEAN PATENT SPECIFICATION

(11) **EP 0 958 301 B1**
(45) Date of publication and mention of the grant of the patent: **23.06.2004**
(21) Application number: 98903762.7
(22) Date of filing: 28.01.1998
(51) Int. Cl.: C07H 13/00

(54) **PURIFICATION OF POLYOL FATTY ACID POLYESTERS USING A MIXING VESSEL WITH CONTROLLED MIXING**
REINIGUNG VON POLYOL-FETTSÄUREPOLYESTER UNTER VERWENDUNG EINES RÜHRGEFÄSSES MIT GESTEUERTEN MISCHUNGSBEDINGUNGEN
PURIFICATION DE POLYESTERS D'ACIDE GRAS DE POLYOL AU MOYEN D'UN RECIPIENT DE MELANGE A MELANGE REGULE

(30) Priority: 31.01.1997 US 36694 P
(43) Date of publication of application: 24.11.1999
(73) Proprietor: THE PROCTER & GAMBLE COMPANY, Cincinnati, Ohio 45202 (US)
(72) Inventor: KENNEALLY, Corey, James, Maineville, OH 45039 (US); SARAMA, Robert, Joseph, Loveland, OH 45140 (US); CLAY, Reginald, Sebastian, Cincinnati, OH 45232 (US); HOWIE, John, Keeney, Oregonia, OH 45054 (US)
(74) Representative: Samuels, Lucy Alice
(86) International application number: PCT/US1998/001557
(87) International publication number: WO 1998/033804

(56) References cited:
- EP-A- 0 319 091
- EP-A- 0 319 092
- EP-A- 0 435 364
- US-A- 4 334 061
- US-A- 4 954 621

## Description

### TECHNICAL FIELD

This invention relates to purification processes for polyol fatty acid polyester, which processes employ a wash solution in a mixing vessel with controlled mixing. Additionally, this invention relates to the purified polyol fatty acid polyester products resulting from the purification processes described herein.

### BACKGROUND OF THE INVENTION

The food industry has recently focused considerable attention on the production of polyol fatty acid polyesters for use as low calorie fats in food products. As a result, there is a continuing need for processes which economically and efficiently produce a relatively high purity polyol fatty acid polyester.

To produce a polyol fatty acid polyester, a polyol can be reacted with a fatty acid lower alkyl ester in the presence of a basic catalyst. In general, polyols are readily soluble in an aqueous medium, e.g. water, while fatty acid lower alkyl esters are soluble in an organic medium. Thus, an emulsifier, solvent, phase transfer catalyst or a mixture thereof is usually required to bring the polyol and the fatty acid lower alkyl ester into physical contact so that they can react chemically. The resulting polyol fatty acid polyester is soluble in an organic medium.

As can be appreciated, the product stream resulting from the reaction of a polyol to produce a polyol fatty acid polyester can therefore contain a variety of components in addition to the desired polyol fatty acid polyester. For example, residual reactants, e.g., unreacted fatty acid lower alkyl ester and/or unreacted polyol, emulsifier, solvent, phase transfer catalyst and/or basic catalyst can be present in the product stream. Additionally, there can be numerous by-products of the reaction itself. For example, numerous side reactions occur in addition to the transesterification of the polyol to form a polyol fatty acid polyester. Side reactions can include the breakdown of one chemical component into two or more by-products, and/or the initial reactants, catalysts, emulsifiers and solvents can chemically react with one another to form undesired by-products, for example, di- and tri-glycerides, beta-ketoesters, di-fatty ketones and unsaturated soaps. Additionally, the initial reactants and other reaction ingredients are often supplied with trace quantities of materials, e.g. trace metals. which are particularly undesirable in a final product which is intended for use as a food additive. Thus. the product stream resulting from the reaction of a polyol and a fatty acid lower alkyl ester can contain, in addition to the desired polyol fatty acid polyester, a variety of undesirable constituents which need to be substantially removed to yield the desired purified polyol fatty acid polyester.

Separation processes for purifying reaction product streams of polyol fatty acid polyesters are generally known to the art. U.S. Patents Nos. 4,241,054, 4,517,360 and 4,518,771 to Volpenhein briefly discuss separation processes for the purification of a polyol fatty acid polyester reaction product. Volpenhein broadly discloses distillation, washing, conventional refining techniques or solvent extraction for purifying the polyol fatty acid polyester. However, the product stream resulting from the transesterification reaction of a polyol provides unique and challenging problems. For example, polyol fatty acid polyesters are often used as low calorie fats, whereby trace quantities of materials which are not suitable for consumption must be removed, whether or not they affect the product's use in nonfood applications. On the other hand, some of the breakdown products of the initial reaction ingredients, for example the caramelized by-product resulting from the breakdown of a polyol, can be suitable for consumption but impart undesirable color and/or increase the caloric content of the product stream, and are thus preferably removed from the reaction product. The complex and highly variable product stream resulting from the transesterification reaction of a polyol to form a polyol fatty acid polyester presents purification process design problems which are both challenging and unique.

A continuing need exists therefore for improved separation and purification processes to purify a polyol fatty acid polyester reaction product stream, particularly resulting from the transesterification of a polyol. More specifically, it is desirable to provide an economical and efficient separation process which can remove water soluble components, emulsifiers, trace metals and other undesirable impurities from polyol fatty acid polyester product.

US 4334061 discloses a process for recovery of polyol fatty acid polyesters comprising contacting the crude reaction product with an aqueous mixture containing organic solvent and, preferably, alkyl, with agitation. The contact time in the examples is generally at least 40 minutes.

### SUMMARY OF THE INVENTION

Accordingly, it is an object of the present invention to provide improved purification processes for purifying polyol fatty acid polyesters. It is a related object of the present invention to provide improved purified polyol fatty acid polyesters, which can be used as food additives.

The invention is defined in claims 1 and 11.

In one embodiment, the invention is directed to a process for purifying an unrefined polyol fatty acid polyester. The process steps include feeding an unrefined polyol fatty acid polyester into a mixing vessel wherein the unrefined polyol fatty acid comprises a polyol fatty acid polyester, soap and impurities. A wash solution is fed into the mixing vessel, and the unrefined polyol fatty acid polyester and the wash solution are dispersed to produce a mixture. At least a portion of the impurities and soap are transferred from the unrefined polyol fatty acid polyester to the wash solution. The mixture is allowed to settle and is then separated into two phases wherein a first phase comprises a purified polyol fatty acid polyester and the second phase comprises an impurity- and soap-containing wash solution. Another embodiment of the present invention is directed towards a purified polyol fatty acid polyester made according to the processes. described herein.

In another embodiment, the invention is directed to a method of making a purified polyol fatty acid polyester wherein a polyol, a soap and a fatty acid lower alkyl ester are reacted to produce a reaction product comprising a polyol fatty acid polyester, soap and impurities. The method comprises the steps of feeding the reaction product and a wash solution into a mixing vessel and dispersing the reaction product and the wash solution to produce a mixture. The mixture is created by controlled mixing of the wash solution and the unrefined polyol fatty acid polyester which provides superior contact between the two streams and thereby promotes the desired mass transfer of impurities from the unrefined polyol fatty acid polyester to the wash solution without the unwanted formation of stable emulsions. The process further comprises the step of separating, such as by settling, the mixture into two phases, wherein the first phase comprises the purified polyol fatty, acid polyester and a second phase comprises the wash solution, followed by separating the first phase from the second phase.

Another embodiment of the present invention provides for the use of a mixing vessel in conjunction with dispersing the wash solution and the unrefined polyol fatty acid polyester under controlled conditions, to avoid the formation of stable emulsions.

### DETAILED DESCRIPTION

The present invention will now be described in detail with reference to specific embodiments. In accordance with the present processes for producing a purified polyol fatty acid polyester, an unrefined polyol fatty acid polyester (often referred to as "crude" polyol fatty acid polyester) is fed into a mixing vessel. A wash solution is fed into the mixing vessel, and an unrefined polyol fatty acid Polyester and the wash solution are dispersed to produce a mixture of the unrefined polyol fatty acid polyester and the wash solution. The mixture contains droplets having an average diameter in the range of from about 5 µm to about 3000 µm preferably from about 5 µm to about 70 µm and most preferably, from about 5 µm to about 20 µm .When mixing is complete, the mixture settles into two phases due to the general immiscibility between the first phase containing the purified polyol fatty acid polyester and the second phase containing the wash solution, soap and impurities.

All droplet diameters reported herein were measured using a Lasentech scanning laser light detector. More specifically the Lasentech instrument is a focused beam reflectance measurement system which consists of a computer interface, a laser diode, detectors, a 10 meter fiber optic cable, and a measuring probe. The light from the laser diode travels down the fiber optic cable to the probe. The light is focused to a very small point in the probe through a sapphire window into the material of interest. When the light beam passes over a particle, or droplet in this case, light is scattered in the backward direction. This light is collected and is passed back to the field unit where it starts a clock. When the light has passed over the droplet, the backscattering stops and this stops the clock. By knowing the speed of the light beam and the length of the backscattering pulse, the diameter of the droplet can be determined. For a given set of conditions, the average droplet diameter is calculated by dividing the sum of all the diameters by the number of droplets measured.

As will be understood, the unrefined polyol fatty acid polyester can conventionally be produced by the reaction of a polyol with a fatty acid lower alkyl ester. However, the unrefined polyol fatty acid polyester can be provided from any available source or production method employed in the art. The purified polyol fatty acid polyester can be used as. among other things, a low calorie fat in foods and, in fact. the purified polyol fatty acid polyester of the present invention is particularly advantageous for use as a food additive owing to its improved purity.

As used herein, the term "wash solution" is intended to include solvents which. when mixed with an unrefined polyol fatty acid polyester under the process conditions described herein, tend to remove at least a portion of the impurities and soap from the unrefined polyol fatty acid polyester. Thus, a wash solution comprises solvents such as water, methanol, acetone and ethyl acetate. Water and more generally, aqueous based wash solutions are preferred for use in the processes described herein due to availability and cost, but it is understood that other solvents are appropriate for use with the processes and methods described herein if the solvents, when mixed with the unrefined polyol fatty acid polyester under the conditions described herein, remove at least a portion of the impurities from the unrefined polyol fatty acid polyester.

Optionally, the wash solution can contain one or more additives, for example, a chelating agent which chelates with metals present in the unrefined polyol fatty acid polyester. The chelating agent can attach, or "chelate", to a portion of the metals from the polyol fatty acid polyester and be carried with the chelated metals into the wash solution. It is important to note however that metals can be removed without the use of a chelant as demonstrated by Example 4 below. Tri-potassium citrate is a preferred chelating agent of the present invention, although other chelating agents are also appropriate and will be apparent to those skilled in the art. A preferred concentration for chelating agents in the present invention is less than about 5.0% by weight of the wash solution. The wash solution should preferably contain less than about 0.5% impurities by weight, prior to its contact with the unrefined polyol fatty acid polyester. An impurity in the water wash solution includes anything that does not aid in the removal of one or more impurities from the polyol fatty acid polyester. Thus, the wash solution can comprise a solvent which removes impurities from the unrefined polyol fatty acid polyester.

As used herein the term "polyol fatty acid polyester" is intended to include any polyol, as defined herein, which has two or more of its hydroxyl groups esterified with fatty acid groups. Preferably, the polyol has been esterified with four or more fatty acid groups. Preferred polyol fatty acid polyesters include sucrose polyesters having on average at least four, more preferably at least five, ester linkages per molecule sucrose; the fatty acid chains preferably have from about eight to about twenty-four carbon atoms. As used herein, the term "unrefined" polyol fatty acid polyester refers to a composition containing predominantly polyol fatty acid polyester containing "impurities" and/or "soap", as defined below, prior to the processes described herein. The amount and type of impurities and soap will vary depending upon, among other things, the source of the polyol fatty acid polyester and the purification steps, if any, the polyol fatty acid polyester is subjected to before it is fed into the mixing vessel of the present invention.

As used herein, the term "polyol" is intended to include any aliphatic or aromatic compound containing at least two free hydroxyl groups. For example, suitable polyols can be selected from the following classes: saturated and unsaturated, straight and branched chain, linear aliphatics: saturated and unsaturated, cyclic aliphatics including heterocyclic aliphatics; or mononuclear and polynuclear aromatics including heterocyclic aromatics.

As used herein, the term "polyol" is intended to include any aliphatic or aromatic compound containing at least two free hydroxyl groups. Suitable polyols can be selected from the following classes: saturated and unsaturated straight and branch chain linear aliphatics: saturated and unsaturated cyclic aliphatics, including heterocyclic aliphatics; or mononuclear or polynuclear aromatics, including heterocyclic aromatics. Carbohydrates and non-toxic glycols are preferred polyols.

Monosaccharides suitable for use herein include, for example, glucose, mannose, galactose, arabinose, xylose, ribose, apiose, rhamnose, psicose, fructose, sorbose, tagatose, ribulose, xylulose, and erythrulose. Oligosaccharides suitable for use herein include, for example, maltose, kojibiose, nigerose, cellobiose, lactose, melibiose, gentiobiose, turanose, rutinose, trehalose, sucrose and raffinose. Polysaccharides suitable for use herein include, for example, amylose, glycogen, cellulose, chitin, inulin, agarose, zylans, mannan and galactans. Although sugar alcohols are not carbohydrates in a strict sense, the naturally occurring sugar alcohols are so closely related to the carbohydrates that they are also preferred for use herein. Natural sugar alcohols which are suitable for use herein are sorbitol, mannitol, and galactitol.

As used herein, the term "impurities" is intended to include a variety of constituents which are undesirable in the purified polyol fatty acid polyester product of the present invention. As will be understood, a particular component, e.g. a di- or tri-glyceride, can be an innocuous constituent of a polyol fatty acid polyester product for one application, but, on the other hand, can be undesirable, i.e. an impurity, in another application. For example, because both di- and tri-glyceride are caloric-containing fats, their presence in a polyol fatty acid polyester which is intended for use as a low calorie fat can be undesirable, whereby the glycerides would both be considered impurities. Likewise, if the polyol fatty acid polyester is intended for use as a food product, trace amounts of metals would be considered impurities if they are not appropriate for consumption by humans. Items such as breakdown products of an initial reactant which is used to form the polyol fatty acid polyester, for example the caramelized by-product of sucrose, can be both inert and suitable for consumption by an average consumer. However, by-products such as the caramelized by-product of a polyol can add undesirable color and/or adversely affect the viscosity of the polyol fatty acid polyester product. Thus, the breakdown product of the initial reactant can be considered an impurity even though it is generally inert and consumable. "Impurity", as used herein, is intended to include anything other than the desired polyol fatty acid polyester, the soap and the fatty acid lower alkyl esters as discussed in greater detail below.

Due to side reactions which occur simultaneously with the transesterification reaction, the polyol fatty acid polyester product can contain various by products which are also considered impurities. Di-fatty ketones and beta-ketoesters are two groups of reaction by-products which are also generally considered impurities and their removal is desirable and non-aqueous solvents are preferred to effect their removal. Fatty acids are often produced by the hydrolysis of a fatty acid lower alkyl ester. Additionally, unsaturated soaps can be formed by the reaction of methyl ester or fatty acid with a catalyst and aqueous wash solutions are preferred to effect their removal.

Among the many potential impurities in a reaction composition containing polyol fatty acid polyester are components from the reaction(s) used to form a polyol fatty acid polyester. As used herein, the term "reaction component" is intended to include any component suitable for use in the production of polyol fatty acid polyester. Suitable reaction components can include, but are not limited to, reactants such as polyol, lower alkyl fatty acid esters and/or glycerides, emulsifiers, catalysts and mixtures thereof.

Fatty acid lower alkyl ester is often reacted with a polyol to form a polyol fatty acid polyester. Additionally, a stoichiometric excess of fatty acid lower alkyl ester is typically provided to completely esterify the polyol. However, feeding excess quantities of fatty acid lower alkyl ester results in a reaction. product containing an appreciable concentration of residual fatty acid lower alkyl ester. The residual fatty-acid lower alkyl ester remaining in the reaction product is not normally soluble in water which is a preferred solvent of the present invention. Additionally, since the fatty acid lower alkyl ester is a feed stock in the reaction of a polyol to form a polyol fatty acid polyester, it is desirable to collect and recycle the residual fatty acid lower alkyl ester. Thus, fatty acid lower alkyl ester is generally not included. within the meaning of the term "impurities" as defined herein.

Fatty acid lower alkyl ester cannot normally be removed by contact with an aqueous based wash solution alone, although small amounts of both the fatty acid lower alkyl ester and the desired polyol fatty acid polyester can be unavoidably entrained in the wash solution. Fatty acid lower alkyl esters are preferably removed from the polyol fatty acid polyester by thermal evaporation. However, the lower alkyl ester evaporates at a lower temperature than does the polyol fatty acid polyester, and any impurities which have boiling points less than the boiling point of the polyol fatty acid polyester may be evaporated along with the lower alkyl ester. To produce a lower alkyl I ester of sufficient purity for direct recycle into the polyol fatty acid polyester production process it is often desirable to remove as much of the soap and impurities as possible using the methods of the present invention prior to the evaporation step.

Additionally, it is desirable to minimize hydrolysis, which results in the formation of free fatty acid, during the treatment processes described herein. The free fatty acid formed by the hydrolysis of lower alkyl ester is difficult to separate from the lower alkyl ester due to the similarity in their vapor pressures. To avoid excessive hydrolysis it is generally preferred to maintain the mixture of wash solution and unrefined polyol fatty acid polyester at a pH of greater than about 5.5 and to avoid the use of acids. Moreover, residence time in the mixing vessel should be less than about 30 minutes, and preferably less than about 15 minutes. Thus, the methods of the present invention can be utilized to treat polyol fatty acid polyesters and to remove impurities from any excess lower alkyl ester making it more suitable for recycle.

A preferred emulsifier for use in the transesterification reaction of a polyol to form a polyol fatty acid polyester is alkali metal fatty acid soap. As used herein, the term "alkali metal fatty acid soap", or "soap" means the alkali metal salts of saturated and unsaturated fatty acids having from about eight to about twenty four carbon atoms. Accordingly, suitable alkali metal fatty acid soaps include, for example, the lithium, sodium, potassium, rubidium, and cesium salts of fatty acids such as capric, lauric, myristic, palmitic, linoleic, oleic, and stearic acids, as well as mixtures thereof. A mixture of fatty acid derived from soybean oil, sunflower oil, safflower oil, cottonseed oil, palm oil and corn oil is preferred for use herein. An especially preferred alkali metal fatty acid soap is, for example, the potassium soap made from palmitic acid and stearic acid. In addition to alkali metal soap, other emulsifiers such as sucrose fatty acid mono-, di- and tri-esters can be used. Solid mono- and di-glycerides can also be used, although they are less preferred.

While an emulsifier in general, and an alkali fatty acid soap specifically, are often desirable reaction components, they are generally undesirable in the polyol fatty acid polyester product. It is desirable to remove substantially all of the soap from the reaction product prior to the thermal evaporation of excess methyl ester to minimize color degradation during evaporation. Additionally, the presence of soap in substantial quantities, i.e. greater than about 2000 ppm can cause processing difficulties when the wash solution and the unrefined polyol fatty acid polyester are dispersed. It is preferred that the concentration of soap in the unrefined polyol fatty acid polyester be below about 2000 ppm, more preferably, below about 1000 ppm and most preferably, below about 500 ppm, to avoid the formation of stable emulsions when the unrefined polyol fatty acid polyester and the wash solution are dispersed. A preferred method for preliminary soap removal is to add a small amount of water to the unrefined polyol fatty acid polyester to facilitate soap coagulation. Hydration is believed to increase the soap phase's specific gravity which aids in the separation process. The amount of water added depends on the level of soap in the unrefined polyol polyester. The ratio of water to soap on a weight basis is from about 1:1 to about 3:1, more preferably from about 1.2:1 to about 2:1 and most preferably from about 1.5:1 to about 1.8: 1. The coagulated soap is then removed by common separation techniques such as settling, filtration or centrifugation. Additionally, as will be discussed in greater detail below, the treated polyol fatty acid polyester, i.e., the polyol fatty acid polyester leaving the mixing vessel described herein, can be further treated by bleaching and/or filtration to further reduce the level of soap below detection limits, i.e., below about 50 ppm. The level of soap in a polyol fatty acid polyester can be measured by a neutralization titration using HCI, or other strong acid, to a predetermined endpoint.

A "base initiator", also known as a "basic catalyst". is generally used to allow the transesterification reaction of a polyol to form a polyol fatty acid polyester to occur at temperature below the degradation temperature of the polyol. Though basic catalyst is a preferred reaction component, it is generally considered an impurity in the polyol fatty acid polyester product stream. Discussions of the types of basic catalysts and their function in the transesterification of polyols can be found in U.S. Patent No. 3,963,699 to Rizzi et al., and U.S. Patent Nos. 4,517,360 and 4,518,772 to Volpenhein. The basic catalyst is typically a strong base with an affinity for hydrogen and is often referred to as a base initiator because it serves to transform the polyol from a stable molecule to a reactive ion. Thus, the terms "basic catalyst" and "base initiator" are interchangeable as used herein. Specifically, the basic catalyst removes a hydrogen from the polyol molecule resulting in a polyol ion in a reactive state. For example, the basic catalyst converts sucrose to sucrate ion. Preferred basic catalysts are carbonate and methoxide ions, which can be complexed with an alkali or alkaline earth metal, for example, potassium or sodium.

As used herein, the term "phase transfer catalyst" is intended to include all chemical species which can interact with a polyol to form a chemical complex, wherein the complexed polyol can travel from one phase to a second phase, and wherein the uncomplexed polyol would not normally be soluble in the second phase. A phase transfer catalyst, as described herein, is to be distinguished from an emulsifier, e.g., a fatty acid soap, in that an emulsifier is believed to provide a single phase in which both chemical species are soluble, i.e. without the need for chemical complexing. As was the case with the other reaction components discussed above, a phase transfer catalyst and any breakdown product resulting therefrom, white often desirable in the transesterification of a polyol to form a polyol fatty acid polyester, is generally considered an impurity in a polyol fatty acid polyester product.

To reduce the levels of impurities in an unrefined polyol fatty acid polyester, the unrefined polyol fatty acid polyester is preferably treated in a mixing vessel with a wash solution resulting in a "treated polyol fatty acid polyester". Specifically, a wash solution is fed into the mixing vessel along with the unrefined polyol fatty acid polyester. The wash solution can be fed counter current or co-current to the unrefined polyol fatty acid polyester or the two solutions can be premixed prior to being fed into the mixing vessel. As used herein "mixing vessel" includes any conventional tank, column or other process equipment which allows the solutions to contact one another. Single stage columns, multistage columns. batch tanks, static mixers and bubble columns are examples of suitable mixing vessels and other appropriate mixing vessels are known to those skilled in the art.

Multistage columns with agitation are preferred mixing vessels for the processes described herein. Both co-current and counter current are equally practical for multistage columns disclosed herein and they are equally efficient for a given droplet size and soap concentration. However, co-current operation is less efficient than counter current with respect to the use of water, although co-current columns are generally easier to scale-up than counter current columns. Once inside the column, the wash solution and the unrefined polyol fatty acid polyester are agitated creating a mixture which is sufficiently controlled to avoid the formation of stable emulsions.

The mixture of unrefined polyol fatty acid polyester and wash solution in the mixing vessel is preferably maintained at a temperature of from about 20 C to about 100 C. more preferably from about 40 C to about 95 C, and most preferably from about 65 C to about 90 C. The mixing vessel can be operated at subatmospheric, atmospheric or superatmospheric pressures. One benefit to operating at superatmospheric pressure is that the temperature of the mixture can be increased slightly since the increase in pressure raises the boiling point of the constituents. Higher temperatures. can be useful to maximize the solubility of the impurities in the wash solution thus, maximizing the purity of the polyol fatty acid polyester. Thus, the mixing vessel can be operated at higher temperatures without boiling the constituents. The benefits of operating the mixing vessels described herein at reduced or increased pressure. must be weighed against the additional equipment and operational costs required with operating at other than atmospheric conditions. Thus, for purposes of efficiency and economics, it is preferred to operate the mixing vessels described herein at atmospheric pressure.

Often, it is desirable to premix the wash solution and the unrefined polyol fatty acid polyester prior to introducing them into the mixing vessel. The use of one inlet stream comprising a pre-mix of unrefined polyol fatty acid and the wash solution can provide manufacturing convenience and economic advantage over feeding the two streams separately to the mixing vessel.

Additionally, the unrefined polyol fatty acid polyester can be pre-treated to reduce the level of impurities and soap prior to being fed into the mixing vessel. For example, an unrefined polyol fatty acid polyester can be centrifuged to remove greater than about 90% by weight of the impurities and soap originally present therein prior to directing the polyol fany acid polyester to the mixing vessel. As discussed above, it is preferred to remove most of the emulsifier, e.g. soap, if present, from the polyol fatty acid polyester in order to avoid the formation of stable emulsions in the wash solution/polyol fatty acid polyester mixture within the mixing vessel. The amount of impurities and soap actually removed in a centrifuge will depend on, at least, the concentration and type of impurities and soap in the unrefined polyol fatty acid polyester, the centrifuge used and the length of time the unrefined polyol fatty acid polyester is centrifuged.

After the wash solution and the unrefined polyol fatty acid polyester are fed into the mixing vessel, they are dispersed to a level sufficient to produce a shear rate which results in the formation of a mixture and avoids the formation of stable emulsions. The mixture, as discussed above, contains droplets of one solution dispersed in the other solution. The droplets have an average diameter within the range of from about 5 µm to about 3000µm The mixture can contain droplets of wash solution dispersed in the unrefined polyol fatty acid polyester or visa versa, or the mixture can contain droplets of both solutions. The composition of the mixture will largely depend on the mass flow rates of each solution fed into the mixing vessel, as is discussed in greater detail below.

The dispersion will depend on, among other process parameters, the size and design of the mixing vessel, the mass flow rate of the solutions fed into the mixing vessel and the type and amount of agitation. "Agitation", as used herein includes any means for producing the mixture of wash solution and unrefined polyol fatty acid polyester. Agitation can be provided by a variety of commonly used processes and types of equipment. For example, impellers and rotating discs can be used to provide dynamic agitation, while forced gas (i.e., "bubbling"), static mixers and pulsation of the feed stream can provide acceptabte non-dynamic agitation of the mixture in the mixing vessel. Agitation by impellers is preferred for use with the mixing vessels described herein, though it is understood that other methods of agitation are also suitable for use in the claimed methods.

As can be appreciated, when impellers are used for agitation their speed and design are important in promoting mixing and mass transfer of impurities from the unrefined polyol fatty acid polyester to the wash solution. By dispersing the mixture sufficiently to produce a shear rate which avoids the formation of stable emulsions, mass transfer of impurities and soap from the unrefined polyol fatty acid polyester to the wash solution can be optimized. As discussed above, other forms of agitation are appropriate for use. with the present invention as long as the agitation is sufficient to produce a shear rate which avoids the formation of stable emulsions and simultaneously forms a dispersion containing the claimed droplet sizes.

The residence time of the mixture within the mixing vessel is also important in maximizing the extent of mass transfer of impurities from the unrefined polyol fatty acid polyester to the wash solution. Preferred residence times of the mixture in the mixing vessel are preferably within the range of from about 0.5 minutes to about 30 minutes, more preferably, from about 1 minute to about 15 minutes, most preferably, from about 1 minute to about 10 minutes and can be selected depending upon, for example, the concentration of impurities and soap in the unrefined polyol fatty acid polyester being fed into the mixing vessel and the desired level of impurities and soap in the treated product.

If the mixing vessel is a column, the number of stages in the column will necessarily affect the residence time as well as the amount of purification that occurs. Selection of the appropriate number of stages will depend on the height and diameter of the column, flow rates of each stream, and the method and amount of agitation, along with other process parameters. When the unrefined polyol polyester and the wash solution are fed co-currently, it is preferred that the column have from about I stage to about 7 stages. When the unrefined polyol polyester and the wash solution are fed counter current, it is preferred that the column have from about 5 stages to about 25 stages.

Another process parameter which can be varied to improve the mass transfer of impurities from the polyol fatty acid polyester to the wash solution is the amount of wash solution fed into the column. A preferred ratio of the mass feed rate of polyol fatty acid polyester to the mass feed rate of the wash solution is in the range of from about 3:1 to about 50:1, and more preferably, from about 4:1 to 20:1. More specifically, the ratio of the mass feed rate of polyol fatty acid polyester to the mass feed rate of the wash solution fed to a co-current multistage column is preferably in the range of from about 3:1 to about 20:1, and the ratio of the mass feed rate of polyol fatty acid polyester to the mass feed rate of the wash solution fed to a counter current multistage column is preferably in the range of from about 4:1 to about 40:1.

As is discussed above, preferred mixing vessels for use with the present invention are multistage columns with agitation. Multistage columns suitable for use with the present invention include, but are not limited to, rotary disc contractors, Oldshue-Rushton extractors, Scheibel extraction towers, Kuhni towers, and the like. These columns are discussed by Perry, et al. *Chemical Engineers Handbook,* 6th Edition, 1984, pages 21-77 to 21-79, incorporated herein by reference. The columns in Perry et al. are schematically shown with counter current flow. A heavy liquid is fed from the top of a vertical column and removed from the bottom with a light liquid fed near the bottom and extracted near the top. As was discussed above, the two streams of the present invention can be fed counter current, i.e., the streams flow through the column in opposite directions, or co-current, i.e., both streams flow through the column in the same direction. When the two streams are fed at or near the same end of the column, they are normally removed at or near the opposite end of the column.

Baffles can be provided between stages within the column wherein the size and shape of the opening in the baffle is designed to provide the desired residence time within each stage and other process conditions. Likewise, within each stage, an impeller can be provided, and typically the impellers are connected to a single shaft which runs through the column. Thus, one shaft can drive all of the impellers, maintaining the agitation speed relatively constant within different stages. However, as can be appreciated, impellers with independent drive motors and or gears can be provided at individual stages or between stages so that the respective impeller speeds vary from one stage to the next. Agitation speed within the column and within individual stages, the size and shape of the baffle openings separating stages and the number of stages are all design criteria which can be varied to achieve a desired purification.

Multistage columns can be provided with "calming" zones at one or both ends of the column wherein the treated mixture (that is, the resulting mixture following sufficient shear rate and residence time to achieve the desired degree of mass transfer of impurities from the polyol to the wash solution) is not agitated and can separate into two phases. If a calming zone is provided, the two phases can then be separated through the use of two extraction ports, i.e., a first port for extracting the first phase and a second port for extracting the second phase.

Having discussed the various solutions and process equipment suitable for use with the processes described herein, the next step is the post treatment processing of the mixture. As used herein "treated" is intended to mean the process of removing at least a portion of impurities and/or soap from the polyol fatty acid polyester. Hence, "treated polyol fatty acid polyester" means the polyol fatty acid polyester resulting when at least a portion of the impurities and soap have been removed by the processes described herein.

Preferably, the treated mixture is removed from the mixing vessel and allowed to settle and separate due to the forces of gravity into the two phases. Each of the two phases can then be separately removed as a treated polyol fatty acid polyester phase and an impurity- and soap-containing wash solution. Other methods of separation are equally appropriate and can be preferred in certain cases. For example, if time is a major consideration and capital and operational costs are not, the treated mixture can be transferred from the mixing vessel to a centrifuge where it can be separated into a light phase, which will normally comprise the treated polyol fatty acid polyester, and a heavy phase, which will normally comprise the impurity- and soap-containing wash solution. The treated polyol fatty acid polyesters of the present invention preferably have a Lovibond Red Scale value of below about 6, more preferably below about 4, and most preferably below about 2. The lower the Lovibond Red Scale value, the lower the level of color bodies in the polyol. The instrument used to measure color bodies was a Lovibond Automatic Tintometer with a red/yellow calibration standard (2.9 red/12.0 yellow).

The treated polyol fatty acid polyester can contain a small amount of the wash solution along with residual impurities and soap while the wash solution can contain a small amount of the polyol fatty acid polyester and other organic oils. It is preferred that the treated polyol fatty acid polyester contains less than about 1% by weight of the total of impurities and wash solution and less than about 100 ppm soap, and more preferably, less than about 50 ppm soap. It is preferred that the wash solution contain not greater than about 5 weight percent of organic oil. Since residual impurities, soap and wash solution can remain in the treated polyol fatty acid polyester after being treated in the mixing vessel, the treated polyol fatty acid polyester can be further refined in additional purification steps. For example, the treated polyol fatty acid polyester can be vacuum dried to remove wash solution and volatile impurities. Particulate silica can also be contacted with the polyol fatty acid polyester to remove particulate impurities and soap. Preferably, the treated polyol fatty acid polyester is vacuum dried prior to the removal of the excess fatty acid lower alkyl esters so that the concentration of wash solution, soap and impurities is less than 0.1% by weight. Additionally, as was discussed above, thermal evaporation to remove excess fatty acid lower alkyl esters, if any are present, can be employed and is often desirable.

The Detailed Description can be better understood when read in conjunction with the following examples wherein polyol fatty acid polyesters are made and water washed to form purified polyol fatty acid polyester product streams having the concentrations tabulated at the end of each example. In the examples, soap levels are measured using the titration methods discussed above and product color resulting from color bodies is measured using a commercially available Lovibond color analyzer.

### Example I

This example compares a wash process using a multistage column with processes employing a packed column and a single stage agitated mixer-settler, respectively. Removal efficiencies. of soap and water soluble color bodies are compared. While it is necessarily difficult to compare three different pieces of equipment under identical process conditions, in this example residence time for each piece of equipment is held constant at about 10-15 minutes to provide a meaningful comparison. Each piece of equipment is operated at a temperature in the range of from about 75 C to about 85 C. The wash solution is deionized water.

### A: Packed Column

A packed column about 3 inches (7.5 cm) in diameter and about 21 inches (53 cm) in length packed with 3/8 inch (1 cm) glass Rasching® rings, is used. Feed rates of the unrefined polyol polyester and wash solution are about 10 lb/hr (4.5 kg/ hr) each. The mean residence time of the polyol polyester and the wash solution is about 15 minutes. The polyol polyester and the wash solution are fed counter-current, with the wash solution entering the top of the column and the polyol polyester entering the bottom of the column. The contaminated wash solution exits at the bottom of the column and the purified polyol polyester exits at the top. The interface between the wash sotution and the polyol fatty acid polyester phase is near the bottom of the packed column. The measured diameter of the water drops is about 3 mm.

### B. Stirred Flask

A 1 liter round bottom flask, fitted with an agitator having a 2 inch (5 cm) long "half moon" impeller, is used for the mixer-settler. About 200g of unrefined polyol polyester and about 37g of wash solution are added to the flask and mixed in a batch mode for about 10 minutes at about 150 rpm. The wash solution becomes dispersed in the polyol polyester in water drops having an average diameter of about 100 µm. The agitator is turned off. A wash solution phase is allowed to settle at the bottom of the flask below the polyol phase and is decanted from the polyol polyester phase.

### C. Multistage Column

An Oldshue-Rushton column about 3 inches (7.5 cm) in diameter and about 16 inches (40 cm) in length, with seven stages is used as the multistage column. Each stage comprises two disc baffles with an inside diameter of about 1 5/8 inches (4 cm) and a vertical spacing of about ) 1 ½ inches (4 cm). A Rushton turbine impeller with a diameter of about I ½ inches (4 cm) is arranged in the middle of each stage. All impellers are mounted on a common shaft which rotates at a speed of 400 rpm. Flow rates of polyol polyester and wash solution are about 12.5 lb/hr (5.6 kg/hr) and about 3.2 lb/hr (1.4 kg/hr), respectively. The mean residence time of the polyol polyester and the wash solution in the column is about 14 minutes. The polyol polyester and wash solution are fed co-current into the top of the column. The product from the bottom of the column is allowed to settle by gravity into separate purified polyol fatty acid polyester and wash solution phases. The measured diameter of the drops is in the range of from about 5 µm to about 70 µm with an average drop diameter of about 15 µm.

Table I sets forth the washed product's soap content and color measurement for each mixing vessel and shows that the multistage column achieves the best results for removal of soap and color from the polyol polyester.

**TABLE I**

| Polyol Polyester Analysis | | |
|---|---|---|
| | Soap, ppm | Color, AOCS Lovibond red |
| Starting material | 638 | 4.2 |
| Packed column | 500 | 4.0 |
| Mixing flask | 179 | 2.4 |
| Multistage column | 161 | 1.8 |

### Example 2

This example illustrates the effect of agitator rpm on color and soap removal in a multistage column. The same multistage column that is described in Example 1, C, is used for this example, including the same flow rates and flow patterns for the polyol polyester and the wash solution. The column is operated at a temperature in the range of from about 75 C to about 85 C. The starting unrefined material is a polyol polyester mixture comprising polyol polyester, fatty methyl ester, potassium stearate soap, sucrose, and water soluble sucrose by-products, and the wash solution source comprises deionized water. Agitator speeds range from 240 rpm to 600 rpm.

Table II sets forth the treated product's soap content and color measurement for each agitator speed and shows that the level of soap and color transferred from the polyol polyester phase to the wash solution is increased as agitator speed is increased.

**TABLE II**

| Polyol Polyester Analysis | | | |
|---|---|---|---|
| | Soap ppm | Color, AOCS Lovibond red | Droplet Size Distribution |
| Starting material | 638 | 4.2 | |
| 240 rpm | 218 | 1.8 | 15 µm - 3000 µm |
| 400 rpm | 161 | 1.8 | Not Measured |
| 550 rpm | 110 | 1.5 | Not Measured |
| 600 rpm | 98 | 1.3 | 5 µm - 70 µm |

### Example 3

This example illustrates the effect of residence time on color and soap removal in a multistage column. The same multistage column that is described in Example I is used for this example. The column is operated at a temperature in the range of from about 75 C to about 85 C. The agitator speed is set at 500 rpm and the unrefined starting material comprises a mixture of polyol polyester, fatty methyl ester, potassium stearate soap, sucrose, and water soluble sucrose by-products. The wash solution source is 0.5% tripotassium citrate in deionized water. Flow rates of the polyol polyester and the wash solution are about 11.6 lb/hr (5.2 kg/hr) and about 4.1 lb/hr (1.8 kg/hr) respectively. The mean residence time for the polyol polyester and wash solution in the column ranges from 5 minutes to 10 minutes, as the number of mixing stages is varied from 3 to 7, .respectively. The measured diameter of the drops is in the range of from about 5 µm to about 70 µm with an average drop diameter of about 15 µm.

Table III sets forth the soap content and color measurement for each washed product and shows that the level of soap and color transferred from the polyol polyester phase to the wash solution phase increased as the residence time (and the number of mixing stages) were increased.

**TABLE III**

| Polyol Polyester Analysis | | |
|---|---|---|
| | Soap, ppm | Color, AOCS Lovibond red |
| Starting mataial | 638 | 4.2 |
| 5 min, 3 stages | 156 | 2.3 |
| 10 min, 7 stages | 94 | 1.8 |

### ExamPle 4

This example compares a production scale, multistage column to a single stage mixer-settler for removing trace levels of iron and calcium from polyol polyester. Each piece of equipment is operated at a temperature in the range of from about 75 C to about 85 C. The wash solution is deionized water.

### A. Multistage Column

An Oldshue-Rushton column about 20 inches (51 cm) in diameter and about 98 inches (250 cm) in length, with seven stages is used as the multistage column. Each stage comprises two disc baffles with an inside diameter of about 10.25 inches (26 cm) and a vertical spacing of about 10 inches (25 cm). In the middle of each stage is a Rushton turbine impeller having a diameter of about 9.75 inches (25 cm). All impellers are mounted on a common shaft which rotates at a speed of 50-230 rpm. Flow rates of polyol polyester and wash solution are approximately 2650 lb/hr (1200 kg/hr) and 477 lb/hr (210 kg/hr), respectively. The mean residence time of the polyol polyester and wash solution in the column is 2-15 minutes. The polyol polyester and wash solution are fed co-current into the top of the column. The measured size of the water drops is less than 1000 µm. The mixture from the bottom of the column is separated by a disc centrifuge into purified polyol fatty acid polyester and wash solution phases.

### B. Mixer-Settler

An 850 gallon (3300 l) tank, fitted with an agitator with a 24 inches (61cm) impeller is used as the mixer-settler. Approximately 2800 lbs. (1250 kg) of polyol polyester and 500 lbs (220 kg) of deionized water are added to the tank and mixed in a batch mode for about 10 minutes at about 20 rpm. The measured size of the water drops is about 3000 µm. The agitator was turned off, and the wash solution phase settles to the bottom ofthe tank and is decanted from the crude polyol polyester.

Table IV sets forth the levels of calcium, iron and copper for each washed product and illustrates that a multistage column achieves the best results for removing trace levels of calcium and iron from the polyol polyester. The results for the mixer-settler are the average of 8 runs, while the results for the multistage column are the average of 4 runs. For both columns, the unrefined starting material comprises a mixture of polyol polyester. fatty methyl ester, potassium stearate soap, sucrose, and water soluble sucrose by-products and the wash solution comprised deionized water with no chelant.

**TABLE IV**

| Polyol Polyester Analysis | | | |
|---|---|---|---|
| | Calcium, ppm | Iron, ppm | Copper. ppm |
| Mixer-settler | 2.51 | 0.49 | 0.08 |
| Agitated column | 0.27 | 0.13 | 0.07 |

### Example 5

This example illustrates the effect of water washing on the removal of trace levels of iron, copper, calcium, and potassium from unrefined polyol fatty acid polyester. A single mixing vessel with a diameter of 20 inches (5 1.0 cm) and a Rushton turbine impeller with a diameter of 9.75 inches (25.0 cm) is used. The agitator speed is 230 rpm. Flow rates of polyol polyester and wash solution are approximately 2650 lb/hr (1200 kg/hr) and 477 lb/hr (210 kg/hr), respectively. The mean residence time of the polyol polyester and the wash solution in the mixing vessel is 2 minutes. The equipment is operated at a temperature of from about 75 C to about 85 C. The wash solution is deionized water. The diameter of the drops is in the range of from about 5 µm to about 70 µm. The mixture from the bottom of the mixing vessel is separated by disc centrifuge into a treated polyol fatty acid polyester phase and an impurity- and soap-containing wash solution phase.

Table V sets forth the treated polyol fatty acid polyester's mineral content and shows that there is a significant reduction from levels in the unrefined polyol polyester.

**TABLE V**

| Polyol Polyester Analysis | | | | |
|---|---|---|---|---|
| | Iron, ppm | Copper, ppm | Calcium, ppm | Potassium, ppm |
| Starting material | 0.58 | 0.14 | 0.44 | 20.41 |
| After water wash | 0.17 | 0.06 | 0.35 | 0.77 |

Having shown and described the preferred embodiments of the present invention, further adaptation of the processes can be accomplished by appropriate modifications by one of ordinary skill in the art without departing from the scope of the present invention. A number of alternatives and modifications have been described herein and others wilt be apparent to those skilled in the art. For example, this reaction can be effectively run in a batch reaction process or a continuous reaction process. Additionally, although specific mixing vessels have been described, other mixing vessels can be used to produce the desired purified polyol fatty acid polyester. Likewise, while numerous polyols and fatty acid lower alkyl esters have been disclosed for the reaction mixture as preferred embodiments of the present invention, the constituents can be varied to produce other embodiments of the present invention as desired.

## Claims

1. A process for purifying an unrefined .polyol fatty acid polyester, which process comprises the steps of:
a) providing an unrefined polyol fatty acid polyester which comprises a polyol fatty acid polyester, preferably a sucrose polyester having on average at least four ester linkages per molecule sucrose, impurities and a soap, preferably the soap being present in an amount of less than 2000 ppm, more preferably less than 1.000 ppm:
b) feeding the unrefined polyol fatty acid polyester and a wash solution into a mixing vessel, preferably at a ratio of mass rate of feeding the unrefined polyol fatty acid polyester to mass rate of feeding the wash solution into the mixing vessel in the range of from 50:1 to 3: 1.
c) dispersing the wash solution and the unrefined polyol fatty acid polyester to produce a mixture containing droplets having an average diameter in the range of from 5 µm to 3,000 µm, preferably in the range of from 5 µm to 70 µm, and for a period of time sufficient for at least a portion of the impurities from the unrefined polyol fatty acid polyester to be transferred to the wash solution, which is less than 30 minutes, preferably less than 15 minutes; and
d) separating the mixture into a first phase comprising treated polyol fatty acid polyester and a second phase comprising impurity- and soap-containing wash solution.

2. The process according to claim 1, further comprising the step of removing the mixture from the mixing vessel before separating it into two phases.

3. The process according to claim 1 or 2, wherein the step of dispersing produces a shear rate sufficient to form the mixture and to avoid the formation of stable emulsions.

4. The process according to any of claims 1 to 3, wherein the wash solution fed into the mixing vessel comprises deionized water, less than 5.0% by weight of a chelating agent and less than 0.5% by weight impurities.

5. The process according to any of claims 1 to 4, wherein the mixing vessel is a static mixer, a bubble column, an agitated tank, an agitated column or an agitated multistage column.

6. The process according to any of claims 1 to 5, wherein dispersing of the wash solution and the unrefined polyol fatty acid polyester to produce a mixture in the mixing vessel is conducted at a temperature of from 20 C to 100 C, and at atmospheric pressure.

7. The process according to any of claims I to 6. further comprising the step of vacuum drying the treated polyol fatty acid polyester to reduce the concentration of wash solution soap and impurities in the resulting treated polyol fatty acid polyester to a concentration of less than 0.1% by weight, and the step of filtering the treated polyol fatty acid polyester with particulate silica to further reduce the concentration of impurities and soap in the resulting treated polyol fatty acid polyester.

8. The process according to any of claims 1 to 7, further comprising the step of removing the soup from the unrefined polyol fatty acid polyester by adding water to said unrefined polyol polyester and separating out the soap phase before it is transfered to the mixing vessel.

9. The process according to any of claims 1 to 8 wherein the ratio of water to soap on a weight basis is from 1:1 to 3:1, preferably from 1.2:1 to 2:1, most preferably from 1.5:1 to 1.8:1.

10. A process according any of claims 1 to 9, wherein the purified polyol fatty acid polyester comprises a polyol fatty acid polyester and less than 1.0% by weight of the wash solution and impurities.

11. A process for preparing polyol fatty acid polyester, comprising the steps of:
a) reacting a mixture comprising a soap, a polyol, preferably sucrose, and fatty acid lower alkyl ester to produce a reaction product comprising a polyol fatty acid polyester, impurities and soap, preferably the soap being present in the reaction product in a concentration of less than 2000 ppm;
b) feeding the reaction product and a wash solution in a mass ratio of from 50:1 to 3:1, preferably from 20:1 to 4:1, into a mixing vessel;
c) dispersing the wash solution and the reaction product to produce a mixture containing droplets having an average diameter in the range of from 5 µm to 3,000 µm, for a period of time sufficient for at least a portion of the impurities from the reaction product to be transferred to the wash solution, which is less than 30 minutes.
d) separating the mixture into a first phase comprising purified polyol fatty acid polyester and a second phase comprising impurity and soap-containing wash solution; and
e) separating the two phases.

12. A process according to Claim 11 comprising the additional step of preliminary soap removal before the reaction product is fed into a mixing vessel.

13. A process according to claims 11 or 12 wherein the ratio of the soap to the water on a weight basis is from 1:1 to 3:1, preferably from 1.2:1 to 2:1, most preferably from 1.5: to 1.8:1.

## Patentansprüche

1. Verfahren zum Reinigen eines rohen Polyolfettsäurepolyesters, welches Verfahren die Schritte umfasst:
a) Vorsehen eines rohen Polyolfettsäurepolyesters, der einen Polyolfettsäurepolyester, vorzugsweise einen Saccharosepolyester mit durchschnittlich mindestens vier Esterbindungen pro Molekül Saccharose, Verunreinigungen und eine Seife umfasst, wobei die Seife vorzugsweise in einer Menge von weniger als 2.000 ppm, weiter vorzugsweise weniger als 1.000 ppm vorliegt;
b) Einspeisen des rohen Polyolfettsäurepolyesters und einer Waschlösung in einen Mischbehälter, vorzugsweise bei einem Verhältnis der Masseneinspeisungsrate des rohen Polyolfettsäurepolyesters zur Masseneinspeisungsrate der Waschlösung in den Mischbehälter im Bereich von 50:1 bis 3:1:
c) Dispergieren der Waschlösung und des rohen Polyolfettsäurepolyesters zur Erzeugung einer Mischung, enthaltend Tröpfchen mit einem durchschnittlichen Durchmesser im Bereich von 5 µm bis 3.000 µm. vorzugsweise im Bereich von 5 µm bis 70 µm, und über einen ausreichenden Zeitraum, damit mindestens ein Teil der Verunreinigungen aus dem rohen Polyolfettsäurepolyester in die Waschlösung überführt wird, welcher weniger als 30 Minuten, vorzugsweise weniger als 15 Minuten beträgt; und
d) Trennen der Mischung in eine erste Phase, umfassend behandelten Polyolfettsäurepolyester, und eine zweite Phase, umfassend Verunreinigungen und Seife enthaltende Waschlösung.

2. Verfahren nach Anspruch 1, umfassend weiterhin den Schritt des Entfernens der Mischung aus dem Mischbehälter bevor sie in zwei Phasen getrennt wird.

3. Verfahren nach Anspruch 1 oder 2, wobei der Schritt des Dispergierens eine ausreichende Scherrate erzeugt, um die Mischung zu bilden und die Bildung stabiler Emulsionen zu verhindern.

4. Verfahren nach mindestens einem der Ansprüche 1 bis 3, wobei die in den Mischbehälter eingespeiste Waschlösung entionisiertes Wasser, weniger als 5.0 Gew.-% eines Komplexbildners und weniger als 0,5 Gew.-% Verunreinigungen umfasst.

5. Verfahren nach mindestens einem der Ansprüche 1 bis 4, wobei der Mischbehälter ein statischer Mischer, eine Blasensäule, ein gerührter Tank, eine gerührte Säule oder eine gerührte Mehrstufensäule ist.

6. Verfahren nach mindestens einem der Ansprüche 1 bis 5, wobei die Dispergierung der Waschlösung und des rohen Polyolfettsäurepolyesters zur Erzeugung einer Mischung in dem Mischbehälter bei einer Temperatur von 20°C bis 100°C und bei atmosphärischem Druck durchgeführt wird.

7. Verfahren nach mindestens einem der Ansprüche 1 bis 6, umfassend weiterhin den Schritt des Vakuumtrocknens des behandeltenen Polyolfettsäurepolyesters, um die Konzentration an Waschlösung, Seife und Verunreinigungen in dem resultierenden, behandelten Polyolfettsäurepolyester auf eine Konzentration von weniger als 0,1 Gew.-% zu reduzieren, und der Schritt des Filtrierens des behandelten Polyolfettsäurepolyesters mit teilchenförmigem Silica, um die Konzentration von Verunreinigungen und Seife in dem resultierenden, behandelten Polyolfettsäurepolyester weiter zu reduzieren.

8. Verfahren nach mindestens einem der Ansprüche 1 bis 7, umfassend weiterhin den Schritt des Entfernens der Seife von dem rohen Polyolfettsäurepolyester durch Zugeben von Wasser zu dem rohen Polyolpolyester und des Abtrennens der Seifenphase, bevor sie zu dem Mischbehälter überführt wird.

9. Verfahren nach mindestens einem der Ansprüche 1 bis 8, wobei das Verhältnis von Wasser zu Seife auf Gewichtsbasis 1:1 bis 3:1, vorzugsweise 1,2:1 bis 2:1, am meisten bevorzugt 1,5:1 bis 1,8:1, beträgt.

10. Verfahren nach mindestens einem der Ansprüche 1 bis 9, wobei der gereinigte Polyolfettsäurepolyester einen Polyolfettsäurepolyester und weniger als 1,0 Gew.-% der Waschlösung und Verunreinigungen umfasst.

11. Verfahren zur Herstellung eines Polyolfettsäurepolyesters, umfassend die Schritte:
a) Umsetzen einer Mischung, umfassend eine Seife, ein Polyol, vorzugsweise Saccharose, und Fettsäureniederalkylester, zur Erzeugung eines Reaktionsprodukts, umfassend einen Polyolfettsäurepolyester, Verunreinigungen und Seife, wobei die Seife vorzugsweise in dem Reaktionsprodukt in einer Konzentration von weniger als 2.000 ppm vorliegt;
b) Einspeisen des Reaktionsprodukts und einer Waschlösung in einem Massenverhältnis von 50:1 bis 3:1, vorzugsweise 20:1 bis 4:1, in einen Mischbehälter;
c) Dispergieren der Waschlösung und des Reaktionsprodukts zur Erzeugung einer Mischung, enthaltend Tröpfchen mit einem durchschnittlichen Durchmesser im Bereich von 5 µm bis 3.000 µm, über einen ausreichenden Zeitraum, damit mindestens ein Teil der Verunreinigungen aus dem Reaktionsprodukt in die Waschlösung überführt wird, welcher weniger als 30 Minuten beträgt;
d) Trennen der Mischung in eine erste Phase, umfassend gereinigten Polyolfettsäurepolyester, und eine zweite Phase, umfassend Verunreinigungen und Seife enthaltende Waschlösung; und
e) Trennung der zwei Phasen.

12. Verfahren nach Anspruch 11, umfassend den zusätzlichen Schritt der vorausgehenden Seifenentfernung, bevor das Reaktionsprodukt in den Mischbehälter eingespeist wird.

13. Verfahren nach Anspruch 11 oder 12, wobei das Verhältnis der Seife zu dem Wasser auf Gewichtsbasis 1:1 bis 3:1, vorzugsweise 1,2:1 bis 2:1, am meisten bevorzugt 1,5:1 bis 1,8:1, beträgt.

## Revendications

1. Procédé pour purifier un polyester d'acide gras de polyol non raffiné, lequel procédé comprend les étapes consistant à:
a) partir d'un polyester d'acide gras de polyol non raffiné qui comprend un polyester d'acide gras de polyol, de préférence un polyester de saccharose ayant en moyenne au moins quatre liaisons ester par molécule de saccharose, des impuretés et un savon, le savon étant de préférence présent en une quantité inférieure à 2000 ppm, mieux encore inférieure à 1000 ppm;
b) introduire le polyester d'acide gras de polyol non raffiné et une solution de lavage dans un récipient de mélange, de préférence à un rapport du débit massique d'introduction du polyester d'acide gras de polyol non raffiné au débit massique d'introduction de la solution de lavage dans le récipient de mélange dans l'intervalle de 50:1 à 3:1;
c) disperser la solution de lavage et le polyester d'acide gras de polyol non raffiné pour produire un mélange contenant des gouttelettes ayant un diamètre moyen compris dans l'intervalle de 5 µm à 3000 µm, de préférence dans l'intervalle de 5 µm à 70 µm, et pendant une période de temps suffisante pour transférer dans la solution de lavage au moins une partie des impuretés du polyester d'acide gras de polyol non raffiné, période de temps qui est inférieure à 30 minutes, de préférence inférieure à 15 minutes; et
d) séparer le mélange en une première phase comprenant le polyester d'acide gras de polyol traité et une seconde phase comprenant la solution de lavage contenant les impuretés et le savon.

2. Procédé selon la revendication 1, comprenant en outre l'étape consistant à enlever le mélange du récipient de mélange avant de le séparer en deux phases.

3. Procédé selon la revendication 1 ou 2, dans lequel l'étape de dispersion produit une vitesse de cisaillement suffisante pour produire le mélange et éviter la formation d'émulsions stables.

4. Procédé selon l'une quelconque des revendications 1 à 3, dans lequel la solution de lavage introduite dans le récipient de mélange comprend de l'eau désionisée, moins de 5,0% en poids d'un agent chélatant et moins de 0,5% en poids d'impuretés.

5. Procédé selon l'une quelconque des revendications 1 à 4, dans lequel le récipient de mélange est un mélangeur statique, une colonne à bulles, un réservoir agité, une colonne agitée ou une colonne agitée à plusieurs étages.

6. Procédé selon l'une quelconque des revendications 1 à 5, dans lequel la dispersion de la solution de lavage et du polyester d'acide gras de polyol non raffiné pour produire un mélange dans le récipient de mélange s'effectue à une température de 20°C à 100°C et à pression atmosphérique.

7. Procédé selon l'une quelconque des revendications 1 à 6, comprenant en outre l'étape de séchage sous vide du polyester d'acide gras de polyol traité pour réduire la concentration en solution de lavage, en savon et en impuretés du polyester d'acide gras de polyol traité résultant à une concentration inférieure à 0,1% en poids, et l'étape de filtration du polyester d'acide gras de polyol traité avec de la silice particulaire pour réduire davantage la concentration en impuretés et en savon du polyester d'acide gras de polyol traité résultant.

8. Procédé selon l'une quelconque des revendications 1 à 7, comprenant en outre l'étape d'élimination du savon à partir du polyester d'acide gras de polyol non raffiné par addition d'eau audit polyester de polyol non raffiné, et la séparation de la phase de savon avant son transfert dans le récipient. de mélange.

9. Procédé selon l'une quelconque des revendications 1 à 8, dans lequel le rapport de l'eau au savon sur une base pondérale est de 1:1 1 à 3:1, de préférence de 1,2:1 à 2:1, mieux encore de 1,5:1 à 1,8:1.

10. Procédé selon l'une quelconque des revendications 1 à 9, dans lequel le polyester d'acide gras de polyol purifié comprend un polyester d'acide gras de polyol et moins de 1,0% en poids de solution de lavage et d'impuretés.

11. Procédé pour préparer un polyester d'acide gras de polyol, comprenant les étapes consistant à:
a) faire réagir un mélange comprenant un savon, un polyol, de préférence le saccharose, et un ester d'alkyle inférieur d'acide gras, pour produire un produit réactionnel comprenant un polyester d'acide gras de polyol, des impuretés et du savon, le savon étant de préférence présent dans le produit réactionnel en une concentration inférieure à 2000 ppm;
b) introduire le produit réactionnel et une solution de lavage dans un rapport massique de 50:1 à 3:1, de préférence de 20:1 à 4:1, dans un récipient de mélange;
c) disperser la solution de lavage et le produit réactionnel pour produire un mélange contenant des gouttelettes ayant un diamètre moyen dans l'intervalle de 5 µm à 3000 µm, pendant une période de temps suffisante pour transférer au moins une partie des impuretés du produit réactionnel dans la solution de lavage, période de temps qui est inférieure à 30 minutes;
d) séparer le mélange en une première phase comprenant le polyester d'acide gras de polyol purifié et une seconde phase comprenant la solution de lavage contenant les impuretés et le savon; et
e) séparer les deux phases.

12. Procédé selon la revendication 11, comprenant l'étape supplémentaire d'élimination préliminaire du savon avant que le produit réactionnel soit introduit dans un récipient de mélange.

13. Procédé selon la revendication 11 ou 12, dans lequel le rapport du savon à l'eau sur une base pondérale est de 1:1 à 3:1, de préférence de 1,2:1 à 2:1, mieux encore de 1,5:1 à 1,8:1.
